(19) Europäisches Patentamt
European Patent Office
Office européen des brevets

(11) Veröffentlichungsnummer: **0 072 905**
**A1**

(12)
# EUROPÄISCHE PATENTANMELDUNG

(21) Anmeldenummer: 82105745.2

(22) Anmeldetag: 29.06.82

(51) Int. Cl.³: C 07 D 271/06
C 07 D 271/10, D 06 L 3/12
C 07 D 405/12
//C07C109/10, C07C63/72,
C07C83/10

(30) Priorität: 04.07.81 DE 3126464

(43) Veröffentlichungstag der Anmeldung:
02.03.83 Patentblatt 83/9

(84) Benannte Vertragsstaaten:
CH DE FR GB IT LI NL

(71) Anmelder: HOECHST AKTIENGESELLSCHAFT
Postfach 80 03 20
D-6230 Frankfurt/Main 80(DE)

(72) Erfinder: Schinzel, Erich, Dr.
Ostpreussenstrasse 43
D-6238 Hofheim am Taunus(DE)

(72) Erfinder: Martini, Thomas, Dr.
Odenwaldstrasse 5
D-6233 Kelkheim (Taunus)(DE)

(72) Erfinder: Spatzier, Winfried
Kassernstrasse 7
D-6238 Hofheim am Taunus(DE)

(72) Erfinder: Probst, Heinz
Billtalstrasse 3
D-6231 Sulzbach(DE)

(54) **Bis-oxadiazolyl-naphthaline, Verfahren zu deren Herstellung und deren Verwendung.**

(57) Bis-oxadiazolyl-naphthaline der allgemeinen Formel

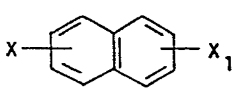

in der X und $X_1$, welche gleich oder verschieden sein können, die Reste

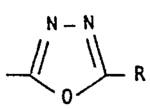 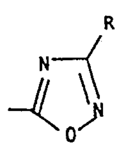 oder 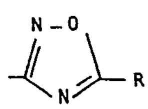

bedeuten, wobei R für Wasserstoff, Alkyl, Alkenyl, Halogenalkyl, Hydroxyalkyl, Alkoxyalkyl, Aryloxyalkyl, Alkylmercaptoalkyl, Arylmercaptoalkyl, niederes Dialkylaminoalkyl, Trialkylammoniumalkyl, Cycloalkyl oder Phenyl, das durch weitere Reste substituiert sein kann, den α- oder β-Naphthylrest, den Benzyl- oder Phenethylrest oder einem Hetarylrest steht und der Naphthalinkern noch weitere nicht farbgebende Reste tragen kann. Diese Verbindungen eingen sich als optische Aufheller.

EP 0 072 905 A1

Croydon Printing Company Ltd

abgewandelte Sulfonsäuregruppen wie Sulfonsäureestergruppen, Sulfonamidgruppen, die am Amidstickstoff noch weitere Substituenten tragen können, niedere Alkyl- oder Arylsulfongruppen, Acylamino-, niedere Dialkylaminoreste, Phenoxy- oder Phenylgruppen substituiert sein kann, den α- oder ß-Naphthylrest, den Benzyl- oder Phenethylrest oder einen Hetarylrest steht und der Naphthalinkern noch weitere nicht farbgebende Reste tragen kann. Bevorzugt sind die Verbindungen der Formel (1) in denen der Naphthalinring in 1,4-Stellung di-substituiert ist.

Besonders bevorzugt sind Verbindungen der Formel (2)

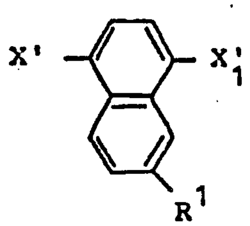

(2)

in der X' und $X_1'$ gleich sind und die Gruppen

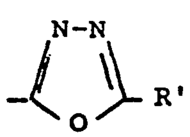

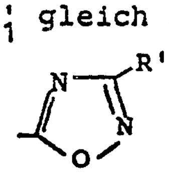

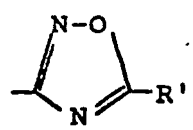

bedeuten, wobei R' Wasserstoff, Alkyl, Benzyl, Benzofuranyl (2) oder eine Gruppe der Formel (3)

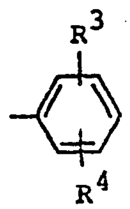

(3)

worin $R^3$ Wasserstoff, Chlor, Cyan, Alkyl, Alkoxy, Carboalkoxy, Carbonamido, Sulfonamido, mono- oder di-Alkylcarbonamido, mono- oder di-Alkylsulfonamido, Alkylsulfonyl oder Phenyl und $R^4$ Wasserstoff, Chlor, Alkyl oder Alkoxy oder $R^3$ und $R^4$ zusammen eine Methylendioxigruppe und $R^1$ Wasserstoff, Halogen, Cyan, Carboalkoxy, Alkylsulfonyl oder Alkoxysulfonyl bedeutet.

--3

HOECHST AKTIENGESELLSCHAFT HOE 81/F 160 0072905

# Bis-oxadiazolyl-naphthaline, Verfahren zu deren Herstellung und deren Verwendung

Mono-oxadiazolyl-naphthaline, die neben dem Oxadiazolylrest im Naphthalinkern noch andere heterocyclische Reste enthalten, sind als Optische Aufheller bereits bekannt. In der DE-OS 2 724 368 werden zum Beispiel 1,2,4-Oxdiazolyl-(5)-naphthaline beansprucht, die als weiteren Substituenten einen Benzofuranyl-(2)-Rest tragen. Die DE-OS 2 748 660 beschreibt 1,2,4-Oxdiazolyl-(5)-Derivate, die sich von Benzoxazolyl-(2)-naphthalinen ableiten.

Gegenstand der Erfindung sind neue Bis-oxadiazolyl-naphthaline der allgemeinen Formel (1)

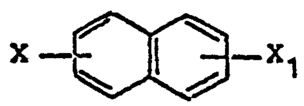 (1)

in der X und $X_1$, welche gleich oder verschieden sein können, die Reste

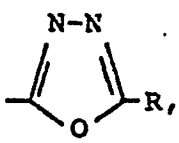, 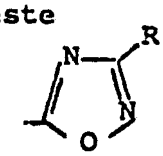 oder 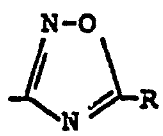

bedeuten, wobei R für Wasserstoff, niederes Alkyl, Alkenyl, Halogenalkyl, Hydroxyalkyl, Alkoxyalkyl, Aryloxyalkyl, Alkylmercaptoalkyl, Arylmercaptoalkyl, niederes Dialkylaminoalkyl, Trialkylammoniumalkyl, Cycloalkyl oder Phenyl, das durch weitere Reste wie Halogen, insbesondere Chloratome, niedere Alkylgruppen, niedere Alkylengruppen, niedere Alkoxygruppen, niedere Alkylendioxygruppen, Carboxygruppen, funktionell abgewandelte Carboxylgruppen wie Cyangruppen, Carbonestergruppen, Carbonamidgruppen, die am Amidstickstoff noch weitere Substituenten tragen können, Sulfogruppen, funktionell

Weiterhin bevorzugt sind die Verbindungen der Formel (2) worin X' und $X_1'$ eine 1,3,4-Oxadiazol-Gruppe bedeuten. Bevorzugt sind auch die Verbindungen der Formel (2), worin $R^1$ Wasserstoff, X' sowie $X_1'$ eine 1,3,4-Oxdiazol-Gruppe und R' eine Gruppe der Formel 3a

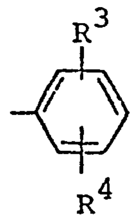

(3a)

worin $R^3$ Wasserstoff, Alkyl, Alkoxy oder Phenyl und $R^4$ Wasserstoff, Alkyl oder Alkoxy bedeutet.

Nichtfarbgebende Substituenten sind vorzugsweise niedere Alkyl-, Alkenyl-, Alkoxy-, Aryl-, vorzugsweise Phenyl-, gegebenenfalls funktionell abgewandelte Carboxy- oder Sulfogruppen, Acyl- Acylamino- oder Sulfonylgruppen sowie Halogenatome. Von den genannten Gruppen können auch mehrere gleichzeitig, die untereinander gleich oder verschieden sind, an den Naphthylenkern gebunden sein.

Die in den oben gegebenen Definitionen enthaltenen Alkyl- und Alkoxygruppen enthalten jeweils 1 bis 6, vorzugsweise 1 bis 4 C-Atome.

Bei den oben angegebenen Definitionen sind unter einer funktionell abgewandelten Carboxylgruppe deren Salze mit farblosen Kationen zu verstehen, wobei Alkalimetall- oder Ammoniumionen bevorzugt sind. Unter Carbonsäureestergruppen sind insbesondere solche der allgemeinen Formel $COOR^2$ zu verstehen, in welcher $R^2$ einen Phenylrest oder eine gegebenenfalls verzweigte niedere Alkylgruppe darstellt, wobei diese Reste weitere Substituenten, wie eine vorzugsweise niedermolekulare Dialkylamino-, Trialkylammonium- oder Alkoxygruppe enthalten können. Unter einer Carbonsäureamidgruppe ist insbesondere eine solche der Formel $CONR^3R^4$ zu verstehen, in welcher die Reste

$R^3$ und $R^4$ Wasserstoffatome oder niedere, gegebenenfalls substituierte Alkylgruppen bedeuten, die auch gemeinsam mit dem Stickstoffatom einen hydroaromatischen Ring bilden können, ferner Säurehydrazide der Formel $CONHNR^3R^4$, in welcher $R^3$ und $R^4$ die obengenannten Bedeutungen haben und die analogen Thioderivate.

Unter einer funktionell abgewandelten Sulfogruppe sind - in Analogie zu den vorstehenden Ausführungen - die Salze mit farblosen Kationen, vorzugsweise Alkalimetall- oder Ammoniumionen, zu verstehen und weiterhin solche Derivate, in denen die $SO_2$-Gruppe an ein Heteroatom gebunden ist, wie in der Sulfonsäureestergruppe und in der Sulfonamidgruppe. Unter Sulfonsäureestergruppe ist insbesondere eine solche der Formel $SO_2OR^2$ zu verstehen, in der $R^2$ die oben angegebene Bedeutung hat, und unter Sulfonsäureamidgruppe eine solche der Formel $SO_2NR^3R^4$, in welcher $R^3$ und $R^4$ die vorstehend angegebenen Bedeutungen haben.

Unter einer Acylgruppe ist insbesondere eine solche der Formel $-COR^5$ zu verstehen, in welcher $R^5$ für einen gegebenenfalls substituierten, vorzugsweise niederen Alkylrest oder Phenylrest, steht.

Unter Sulfonylrest ist insbesondere ein solcher der Formel $SO_2R^6$ zu verstehen, in welcher $R^6$ für eine gegebenenfalls substituierte niedere Alkyl- oder Phenylgruppe steht, wobei diese Gruppe als Substituenten bevorzugt eine niedere Dialkylamino-, Trialkylammonium-, Acylamino- oder Sulfogruppe enthalten können.

Unter denen für R angegebenen Definitionen sind im einzelnen zu verstehen:
Methyl, Äthyl, n- oder i-Propyl, n- oder i-Butyl, Pentyl, ß-Chloräthyl, ß-Dimethyl- oder ß-Diäthylaminoäthyl, N-ß-Morpholinoäthyl, N-ß-Piperidinoäthyl, N-ß-(N'-Methylpiperazino)-äthyl, Phenoxymethyl, ß-Phenoxyäthyl, ß-Chlorphenoxyäthyl, ß-Äthylmercaptoäthyl, ß-

--5

Phenylmercaptoäthyl, oder die Reste der Formeln $CH_2CH_2OCH_3$, $CH_2CH_2OC_2H_5$, $CH_2CH_2OC_3H_7$, $CH_2CH_2OC_4H_9$, $CH_2CH_2OC_6H_{13}$,

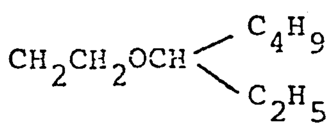, $CH_2CH_2OC_6H_{11}$, $(CH_2CH_2O)_2CH_3$, $(CH_2CH_2O)_2C_2H_5$, $(CH_2CH_2O)_2C_4H_9$, $(CH_2CH_2O)_3C_2H_5$, $CH_2CH_2OCH_2CH_2SC_2H_5$, $CH_2CH_2OCH_2CH_2-N(CH_3)_2$, $CH_2CH_2OCH_2CH_2-N(C_2H_5)_2$ oder $CH_2CH_2O-CH_2CH_2-$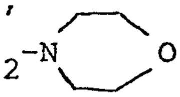

Die erfindungsgemäßen Bis-oxadiazolyl-naphthaline lassen sich nach verschiedenen Methoden synthetisieren. Bevorzugte Verfahren werden im folgenden erläutert.

1. Bis-1,3,4-oxadiazolyl-(5)-naphthaline:

Durch Umsetzung von 1 Mol eines Dicarbonsäurederivates der allgemeinen Formel (4)

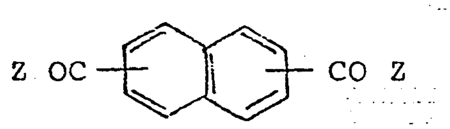 (4)

mit 2 Mol eines Carbonsäurederivats der allgemeinen Formel (5)

$$R - CO\,Z' \qquad (5)$$

wobei in den Formeln (4) und (5) eines der Symbole Z und Z' ein Halogen-, insbesondere Chloratom, und das andere die Gruppe $-NH-NH_2$ bedeuten, in indifferenten organischen Lösemitteln oder Lösemittelgemischen, gegebenenfalls unter Verwendung eines Säureacceptors, beispielsweise einer tertiären Amin-Base wie beispielsweise Pyridin, Triethylamin oder N,N-Dimethylanilin, bei Temperaturen zwischen Raumtemperatur und etwa 220°C vorzugsweise 80 - 130° erhält man Diacylbishydrazide der Formel (6),

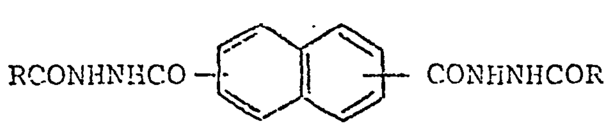 (6)

aus denen man durch Abspaltung von 2 Mol Wasser die erfindungsgemäßen Bis-oxadiazolyl-Derivate der allgemeinen Formel (1) herstellen kann. In den Formeln (5) und (6) besitzen die Reste R die bei der allgemeinen Formel (1) genannten Bedeutungen.

Die unter Wasserabspaltung verlaufenden Oxadiazol-Ringschlüsse führt man entweder mit wasserentziehenden Mitteln, wie z.B. Thionylchlorid, Phosphoroxychlorid oder Polyphosphorsäure, bei Temperaturen zwischen etwa 50°C und etwa 220°C gegebenenfalls in organischen Lösungsmitteln durch oder man erhitzt die Diacylhydrazide der allgemeinen Formel (6) in indifferenten Lösemitteln oder Lösemittelgemischen in Gegenwart von sauren Katalysatoren, wie z.B. organischen Sulfonsäuren, Lewis-Säuren oder Mineralsäuren, insbesondere p-Toluolsulfosäure, Borsäure oder $ZnCl_2$.

Als indifferente Lösemittel dienen unter anderen hochsiedende aromatische oder aliphatische, gegebenenfalls halogenierte, Kohlenwasserstoffe, beispielsweise Trichlorbenzole, Dichlorbenzole, Chlorbenzol, Xylole, Toluol, usw., oder hochsiedende Ester aromatischer Carbonsäuren, wie Benzoesäuremethylester. Gewöhnlich werden die Ringschlußreaktionen bei Temperaturen zwischen etwa 150°C und etwa 250°C durchgeführt.

2. Bis-1,2,4-oxadiazolyl-(3 bzw. 5)-naphthaline:

1 Mol eines Dicarbonsäurederivates der allgemeinen Formel (7)

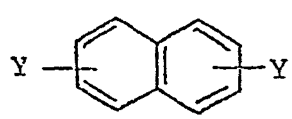

(7)

werden mit 2 Mol eines Carbonsäurederivates der allgemeinen Formel (8)

R - Y' (8)

0072905

wobei in den Formeln (7) und (8) eines der Symbole Y und Y' eine Carbonsäurehalogenid-, insbesondere eine Carbonsäurechloridgruppe und das andere die Amidoxim-Gruppe $-\underset{\substack{| \\ NH}}{C}=N-OH$ bedeuten, gegebenenfalls unter Verwendung eines Säureacceptors, bei Temperaturen zwischen 0° und etwa 60° umgesetzt und aus den gebildeten Acylierungsprodukten gegebenenfalls unter Verzicht auf eine Zwischenisolierung, 2 Mol Wasser abgespaltet. Dieser Ringschluß kann durch bloßes Erhitzen auf 100 - 200°, bevorzugt bei 160 - 200° durchgeführt werden.

In der Formel (8) hat R die unter der Formel (1) angegeben Bedeutung.

Als Lösungsmittel für die Reaktion eignen sich z.B. Äthylenchlorid, Chlorbenzol, Di- oder Trichlorbenzol und insbesondere Dimethylformamid, N-Methylpyrrolidon, Dimethylsulfoxid oder Nitrobenzol. Säurebindende Mittel sind beispielsweise $Na_2CO_3$, $K_2CO_3$, $CaCO_3$ oder tert. Amine, beispielsweise Pyridin, Triethylamin oder N,N-Dimethylanilin. Nach ein- bis zweistündiger Reaktionszeit entstehen unter Abspaltung von HCl und $H_2O$ direkt die Verbindungen der allgemeinen Formel (1), die beim Abkühlen des Reaktionsgemisches in guter Ausbeute und nahezu rein auskristallisieren.

Als Dicarbonsäuren, die den Bis-halogeniden und Bishydroxiden (4) bzw. den Bis-amidoximen (7) zugrunde liegen, können z.B. verwendet werden:

Naphthalindicarbonsäure-(1,2)
" -(1,3)
" -(1,4)
" -(1,5)
" -(1,6)
" -(1,7)
" -(2,3)
" -(2,6)
" -(2,7)

--8

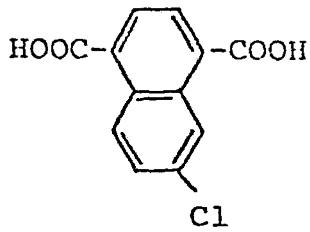
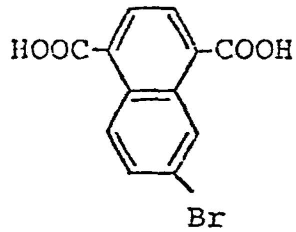
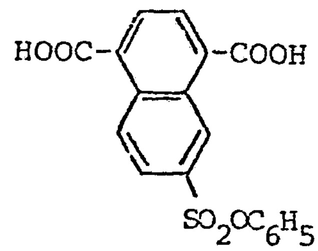
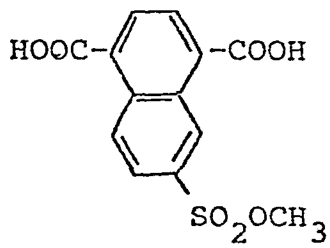
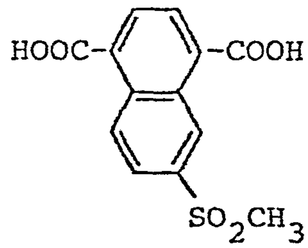
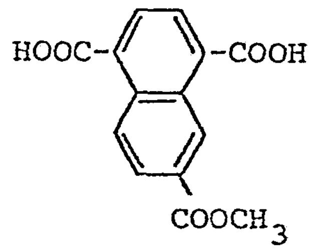
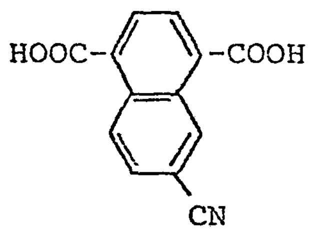

Als Carbonsäuren, die den Carbonsäurederivaten (5) bzw. (8) zugrunde liegen können, seien beispielsweise genannt:

Ameisensäure, Essigsäure, Propionsäure, n-Buttersäure, n-Valeriansäure, Chloressigsäure, ß-Chlorpropionsäure, Bromessigsäure, Pivalinsäure, Ethoxyessigsäure, Acrylsäure, Crotonsäure Tiglinsäure, Methacrylsäure, ß-ß-Dimethyl-acrylsäure, Dichloressigsäure, 2-Brom-propionsäure, 2-Brom-myristinsäure, Glykolsäure, Milchsäure, Cyclohexan-carbonsäure, Dimethylaminoessigsäure, Benzoesäure, Phenylessigsäure, Toluol-2-carbonsäure, Toluol-3-carbonsäure, Toluol-4-carbonsäure, 3-Phenylpropionsäure, 1,4-Xylol-2-carbonsäure, Zimtsäure, Terephthalsäure-monoethylester, 2-Chlor-benzoesäure, 3-Chlor-benzoesäure, 4-Chlor-benzoesäure, 4-Brombenzoesäure, 2,4-Dichlor-benzoesäure, 2,5-Dichlorbenzoesäure, 2-Chlor-toluol-4-carbonsäure, 4-Chlortoluol-3-carbonsäure, 2-Chlor-zimtsäure, 2-Methoxybenzoesäure, 3-Methoxy-benzoesäure, 4-Ethoxy-benzoesäure,

4-Chlor-2-methoxy-benzoesäure, 3,4-Methylen-dioxybenzoesäure, Indan-5-carbonsäure, 1,2,3,4-Tetrahydronaphthalin-6-carbonsäure, Benzoesäure-4-sulfamid, Benzoesäure-3-sulfamid, 4-Phenoxy-benzoesäure, Diphenyl-4-carbonsäure, α-Naphtholsäure, Benzofuran-2-carbonsäure, 2-Diethylamino-benzoesäure, 4-Dimethylamino-benzoesäure, 4-Acetamino-benzoesäure, N-Methyl-diphenylamin-4-carbonsäure, 5-Chlor-2-acetamino-benzoesäure, 3-Acet-amino-4-methoxy-benzoesäure, 5-Chlor-toluol-2-thioglykolsäure, Diphenyl-essigsäure, Naphthyl-(1)-essigsäure, 2-Acetoxy-3-naphthoesäure, Furan-2-carbonsäure, Pyridin-3-carbonsäure, Pyridin-4-carbonsäure.

An den Reaktionsprodukten der vorstehenden Verfahren können natürlich noch weitere an sich bekannte Umwandlungen vorgenommen werden, wie z.B. Sulfonierungen mit sulfonierenden Mitteln, wie beispielsweise $H_2SO_4$, Gemischen aus $H_2SO_4$ und $SO_3$ oder Chlorsulfonsäure, außerdem solche Umwandlungen, die beispielsweise ausgehend von sulfo- oder carboxyhaltigen Molekülen zu Verbindungen mit funktionell abgewandelten Sulfo- oder Carboxygruppen führen bzw. die Umwandlungen solcher Gruppen in andere Gruppen dieser Art oder in die freien Säuren.

Weiterhin können z.B. auch in bekannter Weise Chlormethylgruppen eingeführt oder Methylgruppen oxydiert werden. Ebenso gelingen Halogenierungen und weitere Umsetzungen der eingeführten Halogenatome, so z.B. der Austausch von Chlor oder Brom gegen die -C≡N-Gruppe oder Amin-Funktionen.

Die neuen erfindungsgemäßen Verbindungen haben aufgrund ihres Fluoreszenzvermögens ein weites Anwendungsgebiet. Vor allem dienen sie zum optischen Aufhellen der verschiedensten synthetischen, halbsynthetischen und natürlichen hochmolekularen Materialien.

--10

Unter synthetischen organischen hochmolekularen Materialien sind Polymerisations-, Polykondensations- und Polyadditionsprodukte sowie deren Nachbehandlungsprodukte zu verstehen, z.B.:

Polymerisate auf Basis von α,ß-ungesättigten Carbonsäuren, von Olefin-Kohlenwasserstoffen oder von halogenierten Kohlenwasserstoffen, (wie Polyolefine, Polyvinylchlorid, Polyvinylidenchlorid, Polyacrylnitril u.a.),

Polykondensate auf Basis bi- oder polyfunktioneller Verbindungen mit kondensationsfähigen Gruppen, deren Homo- und Mischkondensationsprodukte (wie Polyester, Polyamide, Maleinatharze, Polycarbonate, Siliconharze u.a.),

Polyadditionsprodukte, wie z.B. vernetzte oder unvernetzte Polyurethane sowie Epoxydharze.

Als halbsynthetische organische Materialien seien z.B. Celluloseester und -äther, Nitrocellulose, regenerierte Cellulose und Kunststoffe auf Casein-Basis genannt.

Optisch aufhellbare natürliche organische Materialien sind z.B. Proteinmaterialien, wie Wolle, Seide und Leder; Cellulosematerialien wie Baumwolle, Papier, Holzmassen in feiner Verteilung; ferner Kautschuk, Guttapercha oder Balata.

Die optisch aufzuhellenden organischen Materialien können in den verschiedensten Verarbeitungszuständen (Rohstoffe, Halbfabrikate oder Fertigfabrikate) und Aggregatzustünden vorliegen, z.B. als Platten, Blätter, Formkörper, Schnitzel, Granulate, Schaumstoffe; Filme, Folien, Lacke, Bänder; Fäden, Fasern z.B. in Form von endlosen Fäden, Stapelfasern, Flocken, Garnen, Strangwaren, Zwirnen, Faservliesen, Filzen, Watten, textilen Geweben, Verbundstoffen und Gewirken; weiterhin auch als Pulver, Kitte, Pasten, Wachse, Kleb- und Spachtelmassen usw.

Die neuen optischen Aufheller können selbstverständlich auch überall dort eingesetzt werden, wo organische Materialien der oben angedeuteten Art mit anorganischen Materialien in irgendeiner Form kombiniert werden.

Die erfindungsgemäßen Verbindungen werden jedoch bevorzugt für die optische Aufhellung von Fasern, Textilien Kunststoffen und Papier verwendet.

Die in Wasser löslichen erfindungsgemäßen anionischen Verbindungen eignen sich besonders zur optischen Aufhellung von nativen und regenerierten Cellulosefasern sowie von Wolle und synthetischen Polyamidfasern.

Die im Wasser löslichen erfindungsgemäßen kationischen Verbindungen eignen sich besonders zur optischen Aufhellung von Homo- und Mischpolymerisaten des Acrylnitrils, insbesondere der handelsüblichen Copolymeren mit einem Mindestgehalt von etwa 85 % Acrylnitrileinheiten.

In Wasser unlösliche erfindungsgemäße Verbindungen, die sich insbesondere für die optische Aufhellung von Polyester- und Polyamidfasern sowie von Cellulose-, Celluloseregeneratfasern und mit Kunstharzen zum Zwecke der Pflegeleichtigkeit ausgerüsteten Cellulose- und Celluloseregeneratfasern allein sowie in Mischung mit Synthesefasern eignen, können gelöst in organischen Lösungsmitteln zum Einsatz kommen oder in wäßriger Dispersion, vorteilhaft unter Zuhilfenahme von Dispergierungsmitteln. Als Dispergierungsmittel kommen beispielsweise Seifen, Polyglykoläther, die sich von Fettalkoholen, Fettaminen oder Alkylphenolen ableiten, Cellulosesulfitablaugen oder Kondensationsprodukte von gegebenenfalls alkylierten Naphthalinsulfonsäuren mit Formaldehyd in Frage.

Die erfindungsgemäßen Verbindungen zeichnen sich insbesondere dadurch aus, daß sie in Gegenwart von oxidativen und reduktiven Bleichmitteln z.B. Wasserstoffperoxid,

Natriumhypochlorit und Natriumchlorit, sowie Natriumdithionit ohne Beeinträchtigung der optischen Aufhellwirkung eingesetzt werden können. Die optischen Aufhellungsmittel können mit anderen Ausrüstungsmitteln zum Zwecke der Effektverbesserung oder der Verfahrensvereinfachung kombiniert werden. Solche Hilfsmittel sind z.B. Retarder, Carrier, Dispergiermittel, Weichmacher, oleophob und hydrophob wirkende Verbindungen, Präparationsmittel, Emulgatoren, Wasch- und Netzmittel. Darüberhinaus weist das aufgehellte Fasermaterial, insbesondere Polyester vom Typ des Polyäthylenglykolterephthalats, eine ausgezeichnete Lichtechtheit auf. Es werden brillante, rot- bis grünstichige Aufhelleffekte mit hohen Weißgraden erzielt.

Besonders gute Aufhelleffekte erhält man zuweilen auch dann, wenn die erfindungsgemäßen Verbindungen mit anderen optischen Aufhellern kombiniert werden. Solche Kombinationen sind insbesondere dann von Interesse, wenn man Nuancenverschiebungen der Aufhelleffekte erzielen will. Ausgezeichnete Effekte werden z.B. bei der Kombination der erfindungsgemäßen Verbindungen mit bekannten Benzoxazolaufhellern der folgenden Konstitutionen erzielt:

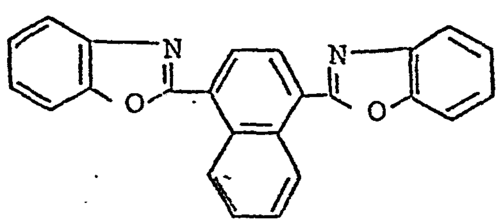

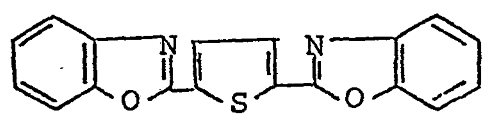

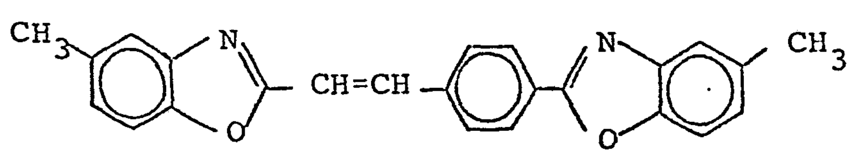

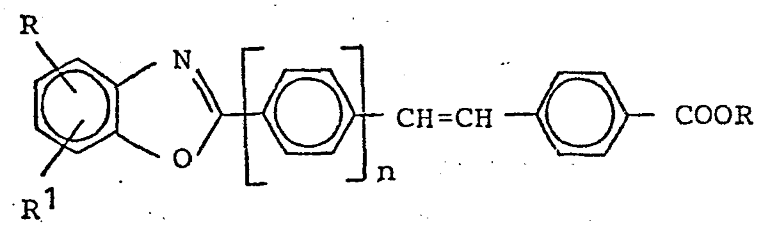

Auch Aufheller vom Cumarintyp, Naphthotriazoltyp, Dialkoxy-triazinyl-pyrentyp, oder Distyrylarene sind als Mischkomponenten gut geeignet.

Die Aufhellung des Fasermaterials mit der wäßrigen oder eventuell organischen Aufhellerflotte erfolgt entweder im Ausziehverfahren bei Temperaturen von vorzugsweise etwa 20 bis 150°C oder unter Thermosolierbedingungen, wobei das Textilmaterial mit der Aufhellerlösung bzw. -dispersion durch Imprägnieren und Abquetschen oder Besprühen auf einen Feuchtigkeitsgehalt von etwa 50 bis 120 % gebracht wird. Anschließend wird das Textilmaterial etwa 10 bis etwa 3000 Sekunden einer Temperaturbehandlung, vorzugsweise mittels Trockenhitze bei etwa 120 bis etwa 240°C unterzogen. Dieser Thermosolierprozeß kann auch mit anderen Ausrüstungsoperationen, z.B. der Ausrüstung mit Kunstharzen zum Zwecke der Pflegeleichtigkeit, kombiniert werden. Die erfindungsgemäßen Aufheller zeichnen sich durch hohe Beständigkeit gegenüber den hierbei üblichen Katalysatoren und Additiven wie Magnesiumchlorid, Zinknitrat oder auch Polyäthylendispersionen aus.

Bis-oxadiazolyl-naphthaline der allgemeinen Formel (1) können auch Waschmitteln zugesetzt werden. Diese können die üblichen Füll- und Hilfsstoffe, wie Alkalisilikate, Alkali-polyphosphate und -polymetaphosphate, Alkaliborate, Alkalisalze der Carboxymethylcellulose, Schaumstabilisatoren, wie Alkanolamide höherer Fettsäuren oder Komplexbildner wie lösliche Salze der Äthylendiamintetraessigsäure oder Diäthylentriaminpentaessigsäure, sowie

chemische Bleichmittel, wie Perborate oder Percarbonate, enthalten. Sehr gute Ergebnisse werden auch bei Perborathaltigen Waschmitteln in Gegenwart von Perborataktivatoren erhalten. Auch die üblichen, in Waschmitteln verwendeten Desinfektionsmittel beeinträchtigen die Aufhellungswirkungen der erfindungsgemäßen Verbindungen nicht.

Ferner können die erfindungsgemäßen Verbindungen hochmolekularen organischen Materialien vor bzw. während deren Verformung zugesetzt werden. So kann man sie beispielsweise bei der Herstellung von Filmen, Folien, Bändern oder Formkörpern den Preßmassen beifügen oder vor dem Verspinnen in der Spinnmasse lösen. Geeignete Verbindungen können auch vor der Polykondensation oder Polymerisation, wie im Falle von Polyamid-6, Polyamid-6,6 oder linearen Polyestern vom Typ des Polyäthylenglykolterephthalats, den niedermolekularen Ausgangsmaterialien zugesetzt werden.

Erfindungsgemäße Verbindungen, die durch eine oder vorzugsweise zwei Carboxy- oder Carboalkoxygruppen substituiert sind, können an lineare Polyestermoleküle und synthetische Polyamide durch eine Ester- oder Amidbindung gebunden werden, wenn sie unter geeigneten Bedingungen diesen Materialien oder bevorzugt deren Ausgangsstoffen zugesetzt werden. Auf diese Weise durch eine chemische Bindung im Substrat verankerte Aufheller zeichnen sich durch eine außerordentlich hohe Sublimier- und Lösungsmittelechtheit aus.

Olefinisch ungesättigte erfindungsgemäße Verbindungen, welche zusätzlich zum fluoreszierenden System mindestens noch eine polymerisierbare olefinische Doppelbindung enthalten, können zur Herstellung von fluoreszierenden Polymerisaten bzw. Polymerisatgemischen verwendet werden, indem man sie unter Erhaltung des fluoreszierenden Systems als solche oder im Gemisch mit anderen monomeren

--15

oder polymeren Vinylverbindungen polymerisiert. Diese fluoreszierenden Polymerisate können anschließend noch mit nicht fluoreszierenden Polymeren vermischt werden. Derartig optisch aufgehellte Polymere zeichnen sich durch einen hohen Weißgrad aus. Darüberhinaus ist aufgrund der chemischen Verankerung der Aufhellermoleküle mit den Polymerisaten eine hohe Sublimier- und Lösungsmittelechtheit gewährleistet.

Die Menge der erfindungsgemäß zu verwendenden Verbindungen der allgemeinen Formel (1), bezogen auf das optisch aufzuhellende Material, kann je nach Einsatzgebiet und gewünschtem Effekt in weiten Grenzen schwanken. Sie kann durch einfache Vorversuche leicht ermittelt werden und liegt im allgemeinen zwischen etwa 0,01 und etwa 2 %.

Die folgenden Beispiele erläutern die Erfindung näher. Die Temperaturen sind in Celsius-Graden angegeben.

Beispiel 1

22,05 g Naphthalindicarbonsäure-(1,4), suspendiert in 200 ml Toluol, werden mit 17,4 ml Thionylchlorid und 2 Tropfen Pyridin versetzt und unter Rührung aufgeheizt. Man hält 1 1/2 Stunden bei 70 - 90° und rührt noch 5 Stunden bei 90 - 105° nach. Anschließend wird durch Abdampfen von 100 ml Lösungsmittel überschüssiges Thionylchlorid entfernt.

27,22 g Benzoesäurehydrazid werden in 150 ml Toluol unter Rühren auf 80° aufgeheizt und bei dieser Temperatur tropfenweise mit obiger Säurechloridlösung versetzt. Unter Durchleiten eines schwachen Stickstoffstroms wird die Temperatur auf 100° gesteigert und in 5 Stunden der abgespaltene Chlorwasserstoff ausgetrieben. Man läßt auf Raumtemperatur erkalten, saugt ab und wäscht mit Toluol und Methanol. Nach dem Trocknen bei 60° im Vakuum

werden 43,73 g des Di-benzoyl-bis-hydroxids der Naphthalin-dicarbonsäure-(1,4) vom Schmelzpunkt 302 - 305° als bräunlich-beiges Pulver erhalten.

22,6 g des Di-benzoyl-bis-hydrazids werden in 300 ml Chlorbenzol suspendiert und nach Zugabe von 18,15 ml Thionylchlorid 24 Stunden unter Rückflußkochen verrührt. Aus der gebildeten, bräunlichen Lösung kristallisiert unter Eiskühlung das Bis-oxadiazol (103) aus, das abgesaugt, mit Ethanol gewaschen und bei 60° im Vakuum getrocknet wird. Man erhält 15,34 g eines hellgelben Kristallpulvers, das nach Umlösung aus Toluol bei 231 - 232° schmilzt.

Beispiel 2

In die nach Beispiel 1, aber unter Verwendung von Chlorbenzol, hergestellte Lösung von 0,05 Mol des Naphthalin-dicarbonsäure-dichlorids-(1,4) werden bei 50 - 60° 15,01 g p-Toluylsäurehydrazid und 12,8 ml N,N-Dimethylanilin eingetragen. Das Reaktionsgemisch wird 5 Stunden bei 100° verrührt, nach dem Abkühlen auf Raumtemperatur mit 100 ml Aceton versetzt und abgesaugt. Man wäscht das Nutschgut mit Aceton und Wasser und trocknet bei 60° im Vakuum. Es werden 22,0 g des Di-toluyl-bis-hydrazids der Naphthalin-dicarbonsäure-(1,4) als weißes Pulver vom Schmelzpunkt 313 - 320° erhalten.

19,22 g des Di-toluyl-bis-hydrazids werden in 250 ml Chlorbenzol mit 14,5 ml Thionylchlorid versetzt und 15 Stunden unter Kühlung am Rückfluß erhitzt, bis die Entwicklung von Chlorwasserstoff beendet ist. Das Reaktionsprodukt wird nach dem Erhalten abgesaugt, mit Aceton gewaschen und im Vakuum bei 60° getrocknet. Man erhält 14,2 g des Bis-oxadiazols (106), das nach Umlösung aus o-Dichlorbenzol ein gelbes Kristallpulver vom Schmelzpunkt 297 - 298° bildet.

Beispiel 3

24,5 g Naphthalin-dicarbonsäure-1,4-bis-hydrazid (Schmp. 251 - 253°) werden unter Stickstoff in 200 ml Toluol mit 16 g Pyridin versetzt und auf 70° erwärmt. Bei dieser Temperatur läßt man in ca. 10 Minuten die aus 39,0 g Monoethyl-terephthalat in 200 ml Toluol mit 16,4 ml Thionylchlorid bereitete Säurechloridlösung zulaufen und erhitzt anschließend 4 Stunden auf 90°. Nach dem Abkühlen wird das Reaktionsprodukt abgesaugt, mit Methanol und Wasser gewaschen und getrocknet. Man erhält 35,4 g des Di-acyl-bis-hydrazids vom Schmelzpunkt 279 - 282° als hellbraunes Pulver.

32,7 g des vorstehenden Reaktionsprodukts werden mit 250 ml Thionylchlorid 7 Stunden unter Kühlung am Rückfluß erhitzt. Anschließend wird das Thionylchlorid abdestilliert, der Rückstand mit einer Mischung aus 150 ml Ethanol und 25 ml konz. Ammoniak verrührt, abgesaugt und mit Ethanol und Wasser gewaschen. Man erhält 28,3 g des Bis-oxadiazols (110), das nach Umlösung aus Chlorbenzol und Dioxan bei 235 - 236° konstant schmilzt.

Beispiel 4

12,7 g Naphthalin-dicarbonsäuredichlorid-(1,4), 17,6 g Cumarilsäurehydroxid und 13 g N,N-Dimethylanilin werden in 250 ml Chlorbenzol 1 1/2 Stunden zum Sieden erhitzt. Anschließend wird auf 0 - 5° abgekühlt, 1 Stunde im Eisbad nachgerührt, abgesaugt, mit Chlorbenzol und Methanol nachgewaschen und getrocknet. Es werden 23,6 g des Di-acyl-bis-hydrazids der Naphthalindicarbonsäure-(1,4) vom Schmelzpunkt 289 - 295° erhalten.

10 g des vorstehenden Produktes werden in 120 ml Trichlorbenzol in Gegenwart von 1 g p-Toluolsulfosäure 1 1/2 Stunden gekocht und gleichzeitig über eine Destillationsbrücke 30 ml Trichlorbenzol abdestilliert. Nach dem Erkalten wird abgesaugt, mit Trichlorbenzol und Ethanol

gewaschen und bei 60° im Vakuum getrocknet. Es werden 7,3 g des Bis-benzolsulfonyl-oxadiazolyl-naphthalins (112) erhalten, das nach Umlösung aus Dimethylformamid einen Schmelzpunkt von 323 - 324° zeigt.

Beispiel 5

Zu einer aus 36,3 g Hydroxylammoniumchlorid und 53,8 ml Acetonitril in Methanol in üblicher Weise bereiteten Lösung von Acetamidoxim wird bei 0 - 5° in 3 1/2 Stunden unter Kühlung die nach Beispiel 1 bereitete Lösung von 0,1 Mol des Naphthalindicarbonsäuredichlorid-(1,4) zugetropft. Die Suspension wird 2 Stunden bei Raumtemperatur nachgerührt. abgesaugt, das Nutschgut mit Methanol gewaschen und getrocknet. Es werden 21,5 g Acylierungsprodukt vom Schmelzpunkt 210 - 217° als beiges Pulver erhalten.

16,4 g des vorstehenden Reaktionsproduktes werden in 82 ml N-Methylpyrrolidon suspendiert und 1/4 Stunde unter Rühren auf 195° erhitzt. Man läßt auf 100° erkalten, filtriert und versetzt das Filtrat mit 200 ml Wasser. Das ausgefallene Bis-oxadiazol wird bei Raumtemperatur abgesaugt, mit Wasser und mit Methanol bis zum farblosen Ablauf gewaschen und im Vakuum bei 60° getrocknet. Es werden 8,6 g des Bis-methyl-oxadiazols (113) erhalten, das nach Umlösung aus Toluol ein bei 173 - 174° konstant schmelzendes, praktisch farbloses Kristallpulver bildet.

Beispiel 6

29,9 g Benzamidoxim (Schmelzpunkt 69°) werden in 200 ml Methanol bei 0 - 5° vorgelegt und tropfenweise mit der nach Beispiel 1 hergestellten toluolischen Lösung des Naphthalindicarbonsäuredichlorids-(1,4) (0,1 Mol) versetzt. Man rührt 3 Stunden bei Raumtemperatur nach und verdampft anschließend das Lösungsmittel. Der harzige Rückstand wird in 100 ml N-Methyl-pyrrolidon aufgenommen und 15 Minuten am Rückfluß gekocht. Beim Abkühlen kristallisiert das Bis-oxadiazol aus, das nach dem Verdünnen mit 100 ml

Methanol und Nachrühren im Eisbad abgesaugt und mit Methanol nachgewaschen wird. Nach dem Trocknen werden 19,5 g des Produktes (114) erhalten, das nach Umlösung aus Toluol bei 206 - 207° schmilzt.

Beispiel 7

12,21 g Naphthalin-dicarbonsäure-1,4-bis-hydrazid (Schmp. 251 - 255°) werden in 50 ml Essigsäureanhydrid eingetragen und nach Zugabe von 4 Tropfen konz. Schwefelsäure unter Kühlung auf 95 - 100° erhitzt. Man hält 3 1/2 Stunden bei dieser Temperatur, läßt auf Raumtemperatur erkalten und saugt das ausgefallene Reaktionsprodukt ab. Nach dem Waschen mit Aceton und Trocknung bei 60° im Vakuum werden 7,4 g eines gebildeten Pulvers erhalten, das nach Umkristallisation aus Dimethylformamid bei 233 - 235° schmilzt und der Konstitution (102) entspricht.

Beispiel 8

12,21 g Naphthalin-dicarbonsäure-1,4-bis-hydrazid (Schmp. 251 - 253°) werden in 150 ml Orthoameisensäurediethylester suspendiert und unter Rühren und Abdestillieren von Ethanol auf eine Innentemperatur von 140° angeheizt. Bei dieser Temperatur werden 100 mg p-Toluolsulfonsäure zugegeben und das Reaktionsgemisch anschließend 100 Stunden am Rückfluß gekocht. Der überschüssige Orthoameisensäureester wird abgedampft, der Rückstand mit 150 ml Wasser versetzt, mit 2n Salzsäure angesäuert und 2 Stunden kräftig durchgerührt. Anschließend saugt man ab, wäscht mit Wasser frei von Chlorionen und trocknet bei 60° im Vakuum. Es werden 11,1 g eines bräunlichen Pulvers erhalten, das sich aus Dimethylformamid reinigen läßt. (101) bildet gelbliche Kristalle, die bei 251 - 253° konstant schmelzen.

Beispiel 9

Zu einer Aufschlämmung von 17,05 g o-Chlor-benzamidoxim

in 50 ml Aceton, welche noch 12,7 ml N,N-Dimethylanilin enthält, läßt man eine Lösung von 0,05 Mol Naphthalin-dicarbonsäure-dichlorid in Toluol (nach Beispiel 1) in 15 Minuten zufließen. Man rührt 6 Stunden bei Raumtemperatur nach, saugt das ausgefallene Reaktionsprodukt ab, wäscht dieses mit Aceton bis zum farblosen Ablauf und dann mit Wasser frei von Chlorionen. Nach dem Trocknen bei 60° im Vakuum erhält man 18,14 g eines beigen Acylierungsproduktes, das bei 206 bis 208° schmilzt. Zum Ringschluß wird dieses Produkt in 50 ml o-Dichlorbenzol suspendiert, mit 30 mg p-Toluolsulfonsäure versetzt und unter Rühren auf 170 - 180° geheizt. Man hält eine halbe Stunde bei dieser Temperatur, kühlt auf Raumtemperatur ab und verdünnt mit 50 ml Methanol. Das Kristallisat wird abgesaugt, mit Methanol gewaschen und bei 60° im Vakuum getrocknet. Es werden 14,7 g der Verbindung (116) in Form hellgelber Kristalle erhalten. Nach dem Umlösen aus o-Dichlorbenzol zeigen diese einen Schmelzpunkt von 211 - 213°C.

Beispiel 10

42,1 g Hydroxyammoniumchlorid werden in 200 ml Glykolmonomethylether aufgeschlämmt und unter Rühren mit 111,72 g einer methanolischen Natriummethylat-Lösung (29,2 %ig) versetzt. Anschließend werden 35,6 g Naphthalin-1,4-dinitril zugegeben. Man erhitzt das Reaktionsgemisch unter Abdestillieren von Methanol auf 115 bis 120° und hält 30 Stunden bei dieser Temperatur. Nach dem Abkühlen auf Raumtemperatur wird das ausgefallene Produkt abgesaugt, mit Wasser frei von Chlorionen gewaschen und bei 60° im Vakuum getrocknet. Das Naphthalin-1,4-bis-amidoxim (42,6 g) bildet ein weißes Pulver, das sich bei 228° zersetzt.

12,2 g des Naphthalin-1,4-bis-amidoxims werden in 150 ml Aceton aufgeschlämmt, mit 12,7 ml N,N-Dimethylanilin versetzt und durch Zutropfenlassen von 11,53 ml Benzoyl-

chlorid benzoyliert. Nach 24-stündigem Nachrühren bei Raumtemperatur wird abgesaugt, mit Aceton und Wasser gewaschen und bei 60° im Vakuum getrocknet. Es werden 18,71 g der weißen Bis-benzoyl-Verbindung erhalten, die bei 227° unter Zersetzung schmilzt.

Der Ringschluß wird wie im vorstehenden Beispiel durchgeführt. Es werden 14,45 g der Verbindung (119) erhalten, die nach Umlösung aus o-Dichlorbenzol einen konstanten Schmelzpunkt von 220 - 222°C zeigt.

Beispiel 11

12,21 g des Naphthalin-1,4-bis-amidoxims (nach Beispiel 10) werden in 30 ml Essigsäureanhydrid suspendiert, mit 2 Tropfen konz. Schwefelsäure versetzt und unter Kühlung auf 105 - 110° angeheizt. Man hält das Reaktionsgemisch 45 Minuten bei dieser Temperatur, kühlt auf Raumtemperatur ab, saugt ab und wäscht mit Aceton. Nach dem Trocknen bei 60° im Vakuum werden 7,0 g eines bräunlichen Pulvers erhalten. Nach dem Umlösen aus Chlorbenzol zeigt (118) einen konstanten Schmelzpunkt von 138 - 140°C.

Tabelle 1

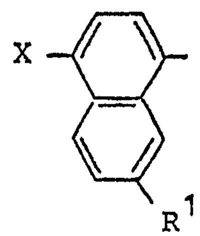

| Lfd.Nr. | X | $R^1$ | Schmelzpunkt (Reinigungsmittel) | Eigenfarbe |
|---|---|---|---|---|
| (101) | 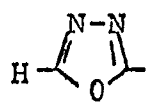 | H | 251 - 253° (Dimethylformamid) | gelblich |
| (102) | 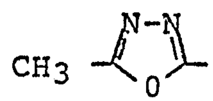 | H | 233 - 235° (Dimethylformamid) | gelblich |
| (103) | 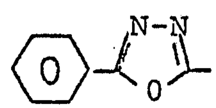 | H | 231 - 232° (Toluol) | hellgelb |
| (104) | 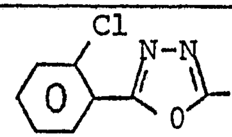 | H | 257 - 258° (o-Dichlorbenzol) | hellgelb |
| (105) | 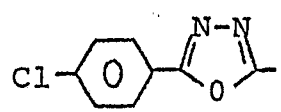 | H | 296 - 297° (Dimethylformamid) | hellgelb |

Fortsetzung Tabelle 1

| Lfd.Nr. | X | $R^1$ | Schmelzpunkt (Reinigungsmittel) | Eigenfarbe |
|---|---|---|---|---|
| (106) | 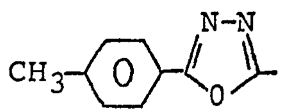 | H | 297 - 298° (o-Dichlorbenzol) | gelb |
| (107) | 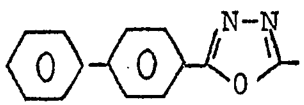 | H | 303 - 305° (o-Dichlorbenzol) | gelblich |
| (108) | 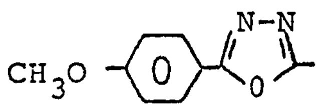 | H | 308,5 - 310° (o-Dichlorbenzol) | gelb |
| (109) | 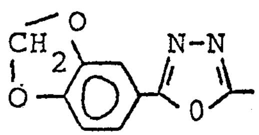 | H | 271 - 273 (o-Dichlorbenzol) | gelb |
| (110) | 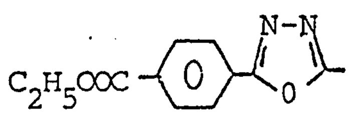 | H | 235 - 236° (1,4-Dioxan) | gelblich |
| (111) | 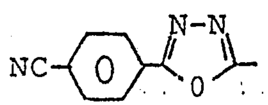 | H | 319 - 321° (o-Dichlorbenzol) | gelblich |

--23

Fortsetzung Tabelle 1

| Lfd.Nr. | X | $R^1$ | Schmelzpunkt (Reinigungsmittel) | Eigenfarbe |
|---|---|---|---|---|
| (112) | 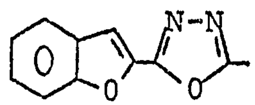 | H | 323 - 324° (Dimethylformamid) | gelb |
| (113) | 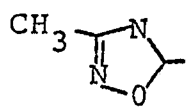 | H | 173 - 174° (Toluol) | farblos |
| (114) | 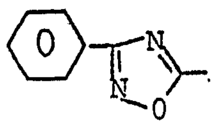 | H | 206 - 207° (Toluol) | gelblich |
| (115) | 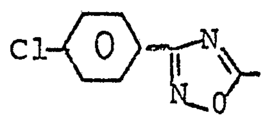 | H | 258 - 260° (o-Dichlorbenzol) | gelblich |
| (116) | 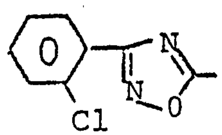 | H | 211 - 213° (o-Dichlorbenzol) | hellgelb |
| (117) | 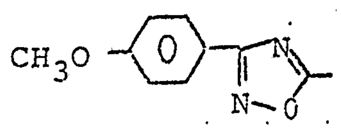 | H | 219 - 220° (o-Dichlorbenzol) | hellgelbe |

Fortsetzung Tabelle 1

| Lfd.Nr. | X | $R^1$ | Schmelzpunkt (Reinigungsmittel) | Eigenfarbe |
|---|---|---|---|---|
| (118) | 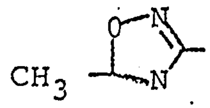 | H | 138 - 140° (Chlorbenzol) | gelblich |
| (119) | 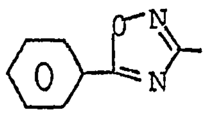 | H | 220 - 222° (o-Dichlorbenzol) | hellgelb |
| (120) | 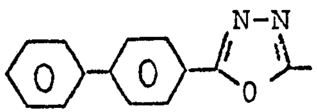 | H | 270 - 272° (Chlorbenzol) | gelb |
| (121) | 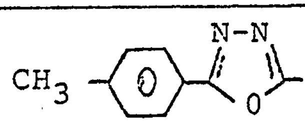 | H | 332 - 335° (1,2,4-Trichlorbenzol) | gelblich |
| (122) | 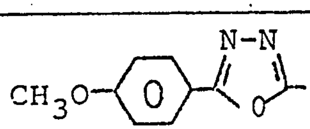 | H | 334 - 338° (1,2,4-Trichlorbenzol) | gelblich |
| (123) | 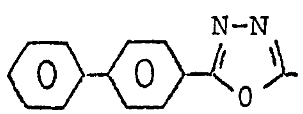 | H | 361 - 363° (1,2,4-Trichlorbenzol) | gelblich |

--25

Beispiel 12

Ein Gewebe aus Polyäthylenterephthalat wurde mit einer Flotte imprägniert, die in dispergierter Form 1 g/l der erfindungsgemäßen Verbindung (107) enthielt. Das so behandelte Textilmaterial wurde zwischen Walzen abgequetscht, bis es nur noch 60 bzw. 100 % seines Trockengewichts an Flüssigkeit enthielt. Nach dem Abquetschen wurde bei 120° 40 Sek. vorgetrocknet und anschließend 40 Sek. bei 180° oder 20 Sek. bei 200° thermosoliert. Das Gewebe wies nach der Behandlung einen ausgezeichneten Weißgrad gegenüber dem unbehandelten Material auf. Außerdem besaß die so aufgehellte Ware eine ausgezeichnete Lichtechtheit von 6 - 7 (nach DIN 54004 geprüft).

Zur Herstellung der Dispersion wurde die Verbindung (107) zunächst in Dimethylformamid (100 mg in 5 ml DMF) unter Erwärmen gelöst, dann mit 5 ml eines 22 bis 25 Ethylenoxideinheiten enthaltenden Nonylphenols versetzt und in dieser Form mit 90 ml Eiswasser verdünnt.

Beispiel 13

Ein Gewebe aus Polyäthylenterephthalat wurde in einem Flottenverhältnis von 1:20 mit einer Waschflotte behandelt, die 6 g/l eines Waschmittels folgender Zusammensetzung enthielt:

| | |
|---|---|
| 50 % | Natriumtripolyphosphat |
| 6 % | Natriummetasilikat |
| 4 % | Carboxymethylcellulose, salzhaltig, Viskosität in 5 %iger Lösung bei 20°C 1500 cP (Höppler-Viskosimeter) |
| 10 % | Isotridecylalkohol - Polyglycoläther (mit durchschnittlich 8 Äthylenglykoleinheiten) |
| 0,05 % | Optischer Aufheller (106) |
| | Rest zu 100 % : $Na_2SO_4$ und Spuren Wasser |

Das Gewebe wurde bei 60° 10 Minuten gewaschen, danach gespült und getrocknet. Diese Behandlung wurde bis zu 10x

wiederholt. Das Gewebe zeigte einen guten Weißgrad und eine deutliche Zunahme des Weißeffektes gegenüber dem nur vorgewaschenen Material (ohne Aufheller).

Beispiel 14

Ein Garn aus Polyäthylenterephthalat wurde im Verhältnis 1:25 in eine Flotte eingebracht, die 0,1 % des Optischen Aufhellers (108) und 0,6 g/l Natriumchlorit enthielt. Der pH-Wert der Flotte wurde mit Ameisensäure auf 3,5 eingestellt. Die kalte Flotte wurde innerhalb 30 Minuten auf 85° aufgeheizt und danach 30 Minuten bei dieser Temperatur gehalten. Nach erneutem Aufheizen auf 120° wurde die Ware 30 Minuten unter diesen Bedingungen weiterbehandelt. Nach dem Spülen und Trocknen wies das Garn einen hervorragenden Weißgrad auf.

Beispiel 15

Ein Gewebe aus Polyamid 6 wurde im Verhältnis 1:30 mit einer Flotte behandelt, die 0,2 % des Optischen Aufhellers (107) enthielt. Der pH-Wert der Flotte wurde mit Oxalsäure auf 4 eingestellt. Die kalte Flotte wurde langsam auf Kochtemperatur erhitzt und das Substrat 30 Minuten darin behandelt. Danach wurde gespült und in üblicher Weise fertiggestellt.
Die so behandelte Ware zeigte einen ausgezeichneten Weißgrad.

Beispiel 16

Ein Gewebe aus Polyäthylenterephthalat wurde im Verhältnis 1:20 in einer Flotte behandelt, die 0,2 % des Optischen Aufhellers (103) und 1 % eines handelsüblichen Carriers auf Basis o-Phenylphenol enthält. Die 50° warme Flotte wurde innerhalb 30 Minuten auf Kochtemperatur aufgeheizt und danach 45 Minuten bei dieser Temperatur behandelt. Anschließend wurde in üblicher Weise gespült und getrocknet. Das Gewebe wies nach der Behandlung einen ausgezeichneten Weißgrad gegenüber dem unbehandelten Material auf.

PATENTANSPRÜCHE:

1. Bis-oxadiazolyl-naphthaline, gekennzeichnet durch die allgemeine Formel 1

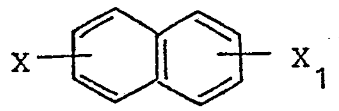

(1)

in der X und $X_1$, welche gleich oder verschieden sein können, die Reste

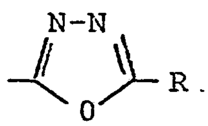

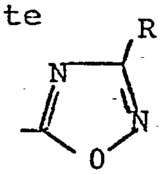

oder

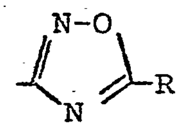

bedeuten, wobei R für Wasserstoff, niederes Alkyl, Alkenyl, Halogenalkyl, Hydroxyalkyl, Alkoxyalkyl, Aryloxyalkyl, Alkylmercaptoalkyl, Arylmercaptoalkyl, niederes Dialkylaminoalkyl, Trialkylammoniumalkyl, Cycloalkyl oder Phenyl, das durch weitere Reste wie Halogen, insbesondere Chloratome, niedere Alkylgruppen, niedere Alkylengruppen, niedere Alkoxygruppen, niedere Alkylendioxygruppen, Carboxylgruppen, funktionell abgewandelte Carboxylgruppen, Sulfogruppen, funktionell abgewandelte Sulfonsäuregruppen, niedere Alkyl- oder Arylsulfongruppen, Acylamino-, niedere Dialkylaminoreste, Phenoxy- oder Phenylgruppen substituiert sein kann, den α- oder ß-Naphthylrest, den Benzyl- oder Phenethylrest oder einen Hetarylrest steht und der Naphthalinkern noch weitere nicht farbgebende Reste tragen kann.

2. Bis-oxadiazolyl-naphthaline nach Anspruch 1, dadurch gekennzeichnet, daß die Gruppen X und $X_1$ in 1,4-Stellun angeordnet sind.

3. Bis-oxadiazolyl-naphthaline nach Anspruch 1 gekennzeichnet durch die allgemeine Formel

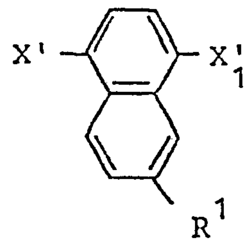

in der X' und $X'_1$ gleich sind und die Gruppen

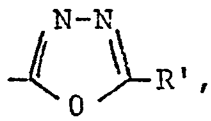,
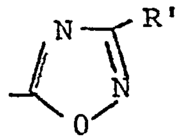 oder 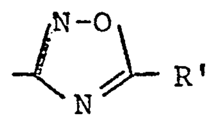

bedeuten, wobei R' Wasserstoff, Alkyl, Benzyl, Benzofuranyl-(2) oder eine Gruppe der Formel 3

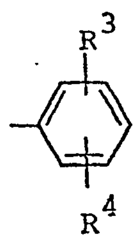 (3)

worin $R^3$ Wasserstoff, Chlor, Cyan, Alkyl, Alkoxy, Carboalkoxy, Carbonamido, Sulfonamido, mono- oder di-Alkylcarbonamido, mono- oder di-Alkylsulfonamido, Alkylsulfonyl oder Phenyl und $R^4$ Wasserstoff, Chlor, Alkyl oder Alkoxy oder $R^3$ und $R^4$ zusammen eine Methylendioxigruppe und $R^1$ Wasserstoff, Halogen, Cyan, Carboalkoxy, Alkylsulfonyl oder Alkoxysulfonyl bedeutet.

4. Bis-oxadiazolyl-naphthaline nach Anspruch 1 gekennzeichnet durch die allgmeine Formel

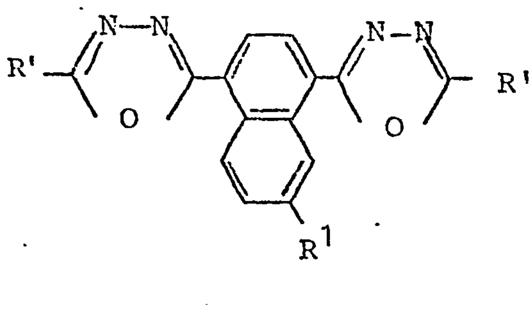

worin R' und $R^1$ die gleiche Bedeutung wie in Anspruch 3 haben.

5. Bis-oxadiazolyl-naphthaline nach Anspruch 1 gekennzeichnet durch die allgemeine Formel

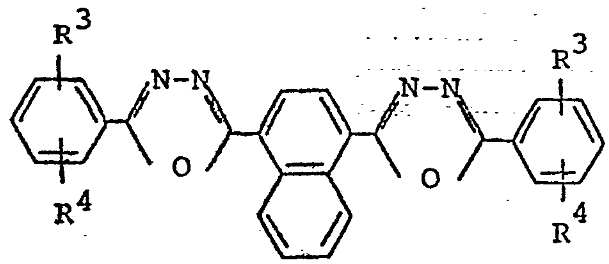

worin $R^3$ Wasserstoff, Alkyl, Alkoxy oder Phenyl und $R^4$ Wasserstoff, Alkyl oder Alkoxy bedeutet.

6. Verfahren zur Herstellung der Bis-oxadiazolyl-naphthaline nach Anspruch 1, dadurch gekennzeichnet, daß man entweder 1 Mol eines Di-carbonsäurederivates der allgemeinen Formel (4)

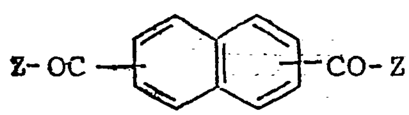 (4)

mit 2 Mol eines Carbonsäurederivats der allgemeinen Formel (5)

R - CO Z' (5)

wobei in den Formeln (4) und (5) eines der Symbole Z und Z' ein Halogen-, insbesondere Chloratom, und das andere die Gruppe $-NH-NH_2$ bedeuten, in indifferenten organischen Lösemitteln oder Lösemittelgemischen, gegebenenfalls unter Verwendung eines Säureacceptors, bei Temperaturen zwischen Raumtemperatur und etwa 220°C zu einem Diacylbishydrazid der Formel (6),

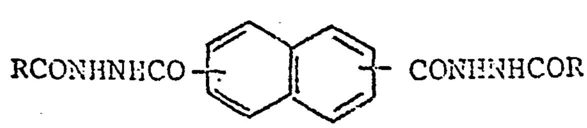 (6)

umsetzt und dieses Diacyl-bis-hydrazid durch Abspalten von 2 Mol Wasser cyclisiert oder 1 Mol eines Dicarbonsäurederivates der allgemeinen Formel (7)

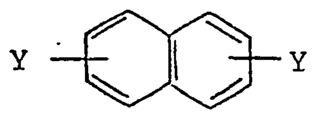

(7)

mit 2 Mol eines Carbonsäurederivates der allgemeinen Formel (8)

$$R - Y' \qquad (8)$$

wobei in den Formeln (7) und (8) eines der Symbole Y und Y' eine Carbonsäurehalogenidgruppe und das andere die Amidoxim-Gruppe $-\underset{NH}{\underset{|}{C}}=N-OH$ bedeuten, in indifferenten organischen Lösemitteln oder Lösemittelgemischen, gegebenenfalls unter Verwendung eines Säureacceptors, bei Temperaturen zwischen 0° und etwa 60° umgesetzt und das gebildete Acylierungsprodukt durch Abspalten von 2 Mol Wasser cyclisiert.

7. Verwendung der Bis-oxadiazolyl-naphthaline nach Anspruch 1 als optische Aufheller.

Europäisches Patentamt

# EUROPÄISCHER RECHERCHENBERICHT

0072905
Nummer der Anmeldung

EP 82 10 5745

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 07 D 271/06 |
| X | DE-A-2 509 514 (HOECHST) * Beispiel 11; Seiten 16-17; Ansprüche * | 1-7 | C 07 D 271/10<br>D 06 L 3/12<br>C 07 D 405/12 //<br>C 07 C 109/10<br>C 07 C 63/72 |
| | --- | | |
| A | DE-A-1 067 439 (BASF) | 1,7 | C 07 C 83/10 |
| | ----- | | |
| | | | **RECHERCHIERTE SACHGEBIETE (Int. Cl. 3)** |
| | | | C 07 D 271/00<br>C 07 D 405/00<br>D 06 L 3/00 |

Der vorliegende Recherchenbericht wurde für alle Patentansprüche erstellt.

| Recherchenort | Abschlußdatum der Recherche | Prüfer |
|---|---|---|
| DEN HAAG | 28-10-1982 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82